# EUROPEAN PATENT APPLICATION

(11) **EP 3 153 146 A1**
(43) Date of publication of application: **12.04.2017**
(21) Application number: 15802927.2
(22) Date of filing: 27.05.2015
(51) Int. Cl.: A61H 1/02, A61B 5/11

(54) **REHABILITATION ASSISTANCE SYSTEM**

(30) Priority: 04.06.2014 JP 2014115856
(71) Applicant: Nihon Kohden Corporation, Shinjyuku-ku Tokyo 161-8560 (JP)
(72) Inventor: MATSUMOTO Hiroyuki, Tokyo 161-8560 (JP); ONO Yoshinobu, Tokyo 161-8560 (JP); YADE Masahiro, Tokyo 161-8560 (JP); MINEGISHI Yuka, Tokyo 161-8560 (JP); SUZUKI Takehito, Tokyo 1618560 (JP)
(74) Representative: Hoffmann Eitle
(86) International application number: PCT/JP2015/065279
(87) International publication number: WO 2015/186586

(57) **Abstract**

A first display apparatus (3) is to be attached to the head (2a) of a patient (2), and enables a body portion of the patient (2) to be seen. A display controller (4) causes an index image that functions as an index for a rehabilitation motion, to be displayed on the first display apparatus (3).

## Description

### Technical Field

The present invention relates to a system for assisting rehabilitation which is performed on a hemiplegia patient after, for example, stroke.

### Background Art

A system is known which is to be used in this kind of rehabilitation, and in which an image of a motion of the patient following instructions is taken, and the motor function of the patient is evaluated by using the taken image (for example, see Patent Literature 1).

### Citation List

### Patent Literature

Patent Literature 1: Japanese Patent No. 5,118,038

### Summary of Invention

### Technical Problem

Based on an example set by a medical person, the patient tries to move a patient's paralyzed limb. Particularly in the initial phase of treatment, however, it is difficult for the patient to move the paralyzed limb following the example, and also to recognize the degree by which the patient's motion is deviated from the target motion. These may cause rehabilitation not to be smoothly progressed.

Therefore, it is an object of the invention to cause the patient to easily know a target motion, thereby enabling rehabilitation to be smoothly progressed.

In order to attain the object, a first mode which the invention may have is a rehabilitation assistance system comprising:
a first display apparatus which is to be attached to a head of a patient, and which enables a body portion of the patient to be seen; and
a display controller which causes an index image that functions as an index for a rehabilitation motion, to be displayed on the first display apparatus.

According to the configuration, an index indicating a motion which is to be performed by the patient can be displayed within the field of view of the patient. Even when the medical person does not set an example, the patient can intuitively understand the motion which is to be performed by the patient, through the index image. From the distance between the patient's motion which can be seen through the first display apparatus, and the index image, moreover, the patient can intuitively understand the degree by which the patient's motion is deviated from the target motion. Therefore, the patient easily recognizes the target motion, and hence rehabilitation can be smoothly progressed.

The index image may contain an image which represents the body portion of the patient.

According to the configuration, the index image is an image which represents a body portion, and therefore the patient can understand more intuitively and naturally the motion which is to be performed by the patient. Consequently, rehabilitation can be smoothly progressed.

The rehabilitation assistance system may further comprise:
a detecting section which detects a motion of the body portion of the patient; and
a storing section which previously stores reference data indicating a target motion of the body portion.

In this case, the display controller changes a color of the index image based on a difference between data indicating the detected motion and the reference data.

According to the configuration, the patient can intuitively understand the degree by which the patient's motion is deviated from the target motion, and moreover the change of the color of the index image becomes incentive to treatment. Therefore, rehabilitation can be smoothly progressed.

The rehabilitation assistance system may further comprise:
a detecting section which detects a motion of the body portion of the patient; and
a recording image producing section which, based on the motion, produces a recording image indicating the motion of the body portion at a first timing.

In this case, the display controller causes the recording image to be displayed on the first display apparatus at a second timing which is later than the first timing.

According to the configuration, when the present rehabilitation is to be performed, the patient can refer the result of rehabilitation which was performed in the past. Therefore, the progress of treatment, i.e., recovery of the motor function can be intuitively known, and as required the recovery can be reflected to the present motion. Consequently, rehabilitation can be smoothly progressed.

The rehabilitation assistance system may further comprise:
a detecting section which detects a motion of the body portion of the patient;
a storing section which previously stores reference data indicating a target motion of the body portion; and
a guiding member which is to be attached to the body portion of the patient, and which outputs at least one of a sound, light, and vibration based on a difference between data indicating the detected motion and the reference data.

According to the configuration, the patient can know more surely a motion which is to be performed by the patient. Therefore, rehabilitation can be smoothly progressed.

The rehabilitation assistance system may further comprise a line-of-sight measurement apparatus which measures a line of sight of the patient.

In this case, the display controller adjusts a position of the index image based on the line of sight.

According to the configuration, a control such as that in which an image for guiding to the index image is displayed on a display area of the first display apparatus in accordance with the measured line of sight can be performed. Therefore, the line of sight of the patient can be easily guided to an adequate position. Consequently, rehabilitation can be smoothly progressed.

The rehabilitation assistance system may further comprise:
a detecting section which detects a motion of the body portion of the patient;
a storing section which previously stores reference data indicating a motion of the body portion, which functions as a reference; and
an evaluating section which numerically evaluates a difference between data indicating the detected motion and the reference data.

According to the configuration, it is possible to quantitatively evaluate the degree by which the patient's motion coincides with the target motion. Therefore, a medical person can quantitatively evaluate the condition of the patient and an effect of rehabilitation without depending on subjectivity of an observer. Consequently, rehabilitation can be smoothly progressed.

The rehabilitation assistance system may further comprise a second display apparatus which is disposed in a place that is separated from a body of the patient.

In this case, the display controller causes a result of evaluation performed by the evaluating section to be displayed on at least one of the first display apparatus and the second display apparatus.

According to the configuration, as required, the result of evaluation performed by the evaluating section can be presented on the side of at least one of the patient and a medical person. Namely, the patient and the medical person can share the evaluation result as required. Therefore, the medical person can express an adequate advice or the like to the patient at an appropriate timing, and sharing of consciousness related to the progress of rehabilitation is prompted. Consequently, rehabilitation can be smoothly progressed.

The display controller may cause an image displayed on the first display apparatus to be displayed also on the second display apparatus.

According to the configuration, the medical person can share the scene as viewed from the viewpoint of the patient, through the second display apparatus. Therefore, the medical person can know easily and intuitively, for example, the difficulty in that the patient follows the target motion. Consequently, the medical person can express an adequate advice or the like to the patient at an appropriate timing, and sharing of consciousness related to the progress of rehabilitation is prompted. Accordingly, rehabilitation can be smoothly progressed.

The rehabilitation assistance system may further comprise a recording section which records a result of evaluation performed by the evaluating section while associating the result of evaluation with at least a timing when the result of evaluation is obtained, and information identifying the patient.

According to the configuration, a database of progress situations of rehabilitation of a patient can be produced. Therefore, an effect of rehabilitation is quantitatively evaluated more easily. Consequently, rehabilitation can be smoothly progressed.

In order to attain the object, a second mode which the invention may have is a rehabilitation assistance system comprising:
a detecting section which detects a motion of a body portion of a patient;
a storing section which previously stores reference data indicating a target motion of the body portion; and
a guiding member which is to be attached to the body portion of the patient, and which outputs at least one of a sound, light, and vibration based on a difference between data indicating the detected motion and the reference data.

According to the configuration, it is possible to cause even a patient in whom a display apparatus is hardly attached to the head, to easily recognize the target motion. Therefore, rehabilitation can be smoothly progressed.

### Brief Description of Drawings

[Fig. 1] Fig. 1 is a diagram illustrating a rehabilitation assistance system of a first embodiment.
[Fig. 2] Figs. 2(a), 2(b) and 2(c) are views illustrating an example of an image which is displayed on a first display apparatus included in the system.
[Fig. 3] Fig. 3 is a view illustrating another example of the image which is displayed on the first display apparatus.
[Fig. 4] Fig. 4 is a view illustrating a further example of the image which is displayed on the first display apparatus.
[Fig. 5] Fig. 5 is a view illustrating a motion of a guiding member included in the system.

### Description of Embodiments

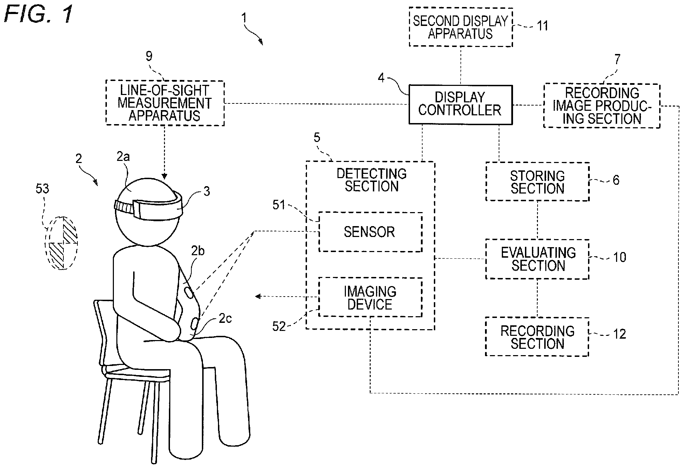

Hereinafter, embodiments will be described in detail with reference to the accompanying drawings. Fig. 1 is a functional block diagram illustrating a rehabilitation assistance system 1 (hereinafter, abbreviated as the assistance system 1) of a first embodiment. The assistance system 1 of the embodiment is configured so as to assist rehabilitation which is performed on a hemiplegia patient 2 after, for example, stroke.

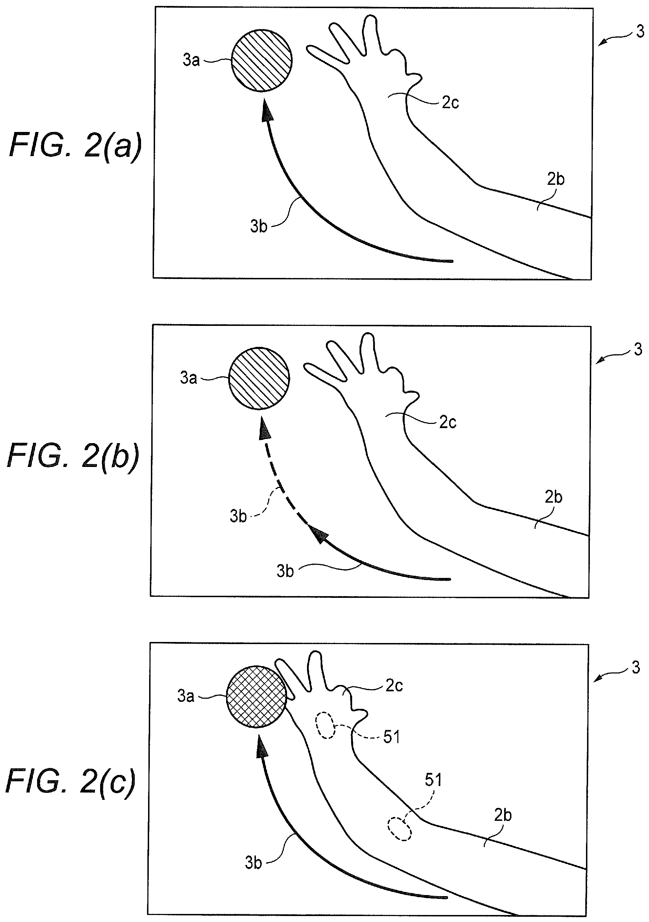

The assistance system 1 includes a first display apparatus 3. The first display apparatus 3 is configured so as to be attached to the head 2a of the patient 2. An example of the first display apparatus 3 is a head-mounted display. The first display apparatus 3 is configured so as to be able to see a body portion of the patient 2. Fig. 2(a) illustrates a manner in which the patient 2 views the left arm 2b and left hand 2c of the patient through the first display apparatus 3. A body portion of the patient may be directly viewed through a transparent screen. Alternatively, the body portion which is imaged by an imaging device disposed in the first display apparatus 3 may be displayed on an opaque screen.

As illustrated in Fig. 1, the assistance system 1 includes a display controller 4. The display controller 4 is configured so as to cause an index image which functions as an index for a rehabilitation motion, to be displayed on the first display apparatus 3. Fig. 2(a) illustrates an example of the index image which is displayed on the first display apparatus 3. The index image of the example contains a target position index 3a and a target locus index 3b.

For example, the target position index 3a is an arbitrary figure (in the example, a circle). The target position index 3a indicates the position where a body portion (in the example, the left hand 2c) of the patient 2 is to be placed. For example, the position is a position which is instructed to the patient 2 by a medical person.

For example, the target locus index 3b is an arrow. The target locus index 3b indicates a locus along which a body portion (in the example, the left arm 2b and the left hand 2c) of the patient 2 is to move. For example, the locus is a locus which is instructed to the patient 2 by the medical person.

In Fig. 2(a), the target locus index 3b is displayed as a still image. However, the target locus index 3b may be provided as a motion image. In an example of the target locus index 3b illustrated in Fig. 2(b), a manner is illustrated in which the arrow indicated by the solid line gradually extends to a position indicated by the broken line, and reaches the state illustrated in Fig. 2(a). Alternatively, the motion image may be displayed in synchronization with a motion of the patient, or asynchronously displayed. For a patient under rehabilitation, particularly, a motion image which is synchronously displayed may function as an index for a rehabilitation motion. For a patient before rehabilitation, by contrast, a motion image which is asynchronously displayed may function as an example of a rehabilitation motion which is to be performed from now.

The positions of the target position index 3a and target locus index 3b which are displayed vary depending on instructions issued by the medical person (i.e., the contents of rehabilitation). When a body portion which is to be treated is viewed through the first display apparatus 3, the position of the body portion in the screen is generally constant, and therefore the position where the index image is to be displayed is naturally determined according to the contents of rehabilitation. The display controller 4 causes the target position index 3a and the target locus index 3b to be displayed at the positions which are previously determined as described above.

According to the configuration, an index indicating a motion which is to be performed by the patient 2 can be displayed within the field of view of the patient 2. Even when the medical person does not set an example, the patient can intuitively understand the motion which is to be performed by the patient, through the index image. From the distance between the patient's motion and index image which can be seen through the first display apparatus 3, moreover, the patient can intuitively understand the degree by which the patient's motion is deviated from the target motion. Therefore, the patient 2 easily recognizes the target motion, and rehabilitation can be smoothly progressed.

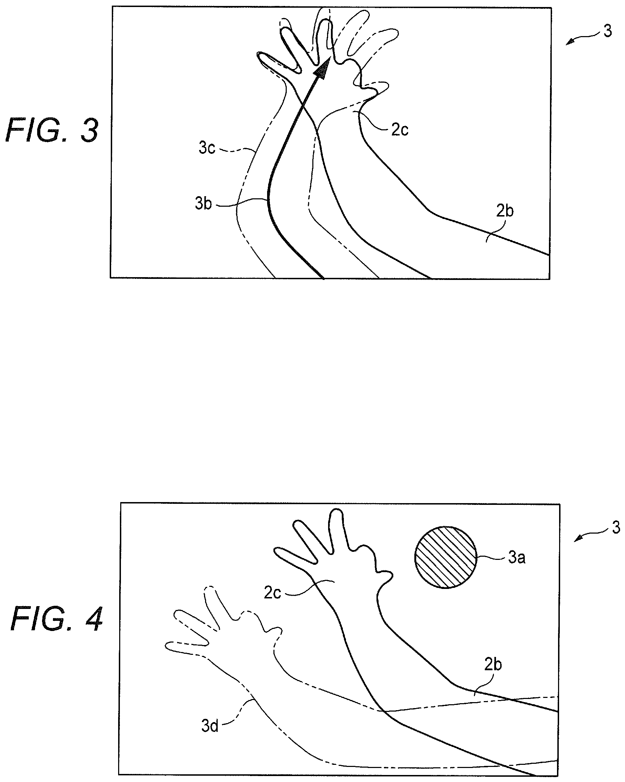

Fig. 3 illustrates another example of the index image which is displayed on the first display apparatus 3. The index image in the example contains an image 3c which represents a body portion (in the example, the left arm and the left hand) of the patient 2. The image 3c may be either a still image or a motion image. The image 3c may be a computer graphics image which is previously prepared by modeling a usual body portion, or an image in which an image that is acquired by previously imaging a body portion of the patient 2 is superimposedly displayed.

According to the configuration, the index image is an image which represents a body portion, and therefore the patient 2 can understand more intuitively and naturally the motion which is to be performed by the patient. Consequently, rehabilitation can be smoothly progressed.

As illustrated in Fig. 1, the assistance system 1 may have a configuration where the system includes a detecting section 5 and a storing section 6. The detecting section 5 is configured so as to detect a motion of a body portion of the patient 2. The detecting section 5 may include at least one of a sensor 51 and an imaging device 52.

The sensor 51 is to be attached to a body portion of the patient 2. For example, the sensor 51 may be an inertial sensor. The inertial sensor is configured so as to output a signal corresponding to the displacement (the velocity, acceleration, and attitude change) of the attachment portion. The attachment portion of the sensor 51 is adequately determined at a body portion where follow-up of recovery of the motor function must be performed, in accordance with the contents of rehabilitation. In the example illustrated in Fig. 1, a plurality of sensors 51 are attached to the left arm 2b and left hand 2c of the patient 2.

The imaging device 52 is configured so as to acquire an image of a body portion of the patient 2. An example of the imaging device 52 is a camera including an imaging device such as a CCD (Charged Coupled Device) sensor or a CMOS (Complementary Metal Oxide Semiconductor) sensor. In order to know the three-dimensional shape of a body portion of the patient 2, the imaging device 52 is configured so as to be able to acquire depth information. Based on the image recognition technique, the imaging device 52 detects the position and motion of a body portion of the patient 2 which is a detection target, in the three-dimensional space. The imaging device 52 is configured so as to output a signal corresponding to the detected position and motion of the target.

When an image is to be acquired by the imaging device 52, preferably, a calibrator 53 is placed in the vicinity of the patient 2 as illustrated in Fig. 1. The calibrator 53 is a member including at least one of a shape, pattern, scale marks in which the dimension is normalized. Here, the term "normalized" means that one unit of the scale marks or the pattern, or the size of the calibrator 53 itself is corresponded to another unit such as 1 cm or 10 inches.

According to the configuration, the imaging device 52 performs image recognition with respect to the calibrator 53, and can correctly specify the motion of the body portion of the patient 2 without using the distance between the imaging device 52 and the patient 2.

The detecting section 5 acquires measurement data indicating the position and motion of the body portion of the patient 2 which is a detection target, in the three-dimensional space based on the signal that is output from at least one of the sensor 51 and the imaging device 52.

The storing section 6 previously stores reference data indicating the target motion with respect to each body portion which will be a treatment target. The reference data have a form corresponding to the measurement data which are acquired by the detecting section 5. In the example, the reference data contain the position and motion of each body portion in the three-dimensional space. Examples of the storing section 6 are a memory element, a large-capacity storage device (such as a hard disk drive), and a portable storage medium (a disk-type storage medium, a card-type storage medium, a USB memory, or the like).

In this case, the display controller 4 may be configured so as to, as illustrated in Fig. 2(c), change the color of the index image which is displayed on the first display apparatus 3, based on the difference between the measurement data of a body portion of the patient 2 which are acquired through the detecting section 5, and the reference data of the body portion that are stored in the storing section 6. The positions of the left arm 2b and left hand 2c of the patient 2 in Fig. 2(c) are closer to the target position index 3a and the target locus index 3b as compared to the example illustrated in Fig. 2(a). In such a case, for example, the color (at least one of the color shade and the color strength) of the target position index 3a and the target locus index 3b is changed to a predetermined color. For example, as the motion of the patient 2 is closer to the target motion, the color of of the index image is made more emphatic. The color may be continuously or stepwisely changed. This color change may be applicable also to the image 3c illustrated in Fig. 3.

According to the configuration, it is possible to intuitively understand the degree by which the patient's motion is deviated from the target motion, and moreover the change of the color of the index image becomes incentive to treatment. Therefore, rehabilitation can be smoothly progressed.

As illustrated in Fig. 1, the assistance system 1 may have a configuration where the system includes a recording image producing section 7. The recording image producing section 7 is configured so as to produce a recording image indicating the motion of the body portion at a first timing, based on the motion of the body portion of the patient 2 which is detected by the detecting section 5. In this case, the display controller 4 causes the recording image produced by the recording image producing section 7, to be displayed on the first display apparatus 3 at a second timing which is later than the first timing. The recording image is a computer graphics image which is prepared by modeling the body portion of the patient 2.

Fig. 4 illustrates an example where a recording image 3d is displayed on the first display apparatus 3. The patient 2 can simultaneously view the recording image 3d which is produced based on the motion in past rehabilitation, and which indicates a body portion of the patient, and the body portion at the current timing. In this example, it is possible to see the manner in which the present motion is closer to the target position index 3a as compared with the past motion.

According to the configuration, when the present rehabilitation is to be performed, the patient 2 can refer the result of rehabilitation which was performed in the past. Therefore, the progress of treatment, i.e., recovery of the motor function can be intuitively known, and as required the recovery can be reflected to the present motion. Consequently, rehabilitation can be smoothly progressed.

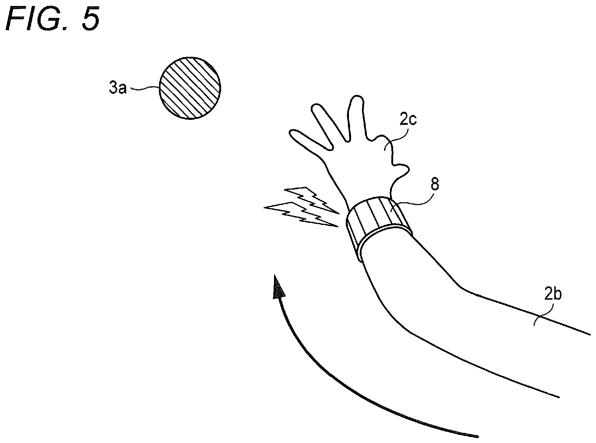

As illustrated in Fig. 5, the assistance system 1 may have a configuration where the system includes a guiding member 8. The guiding member 8 is configured so as to be attached to a body portion (here, the left arm 2b) of the patient 2. The guiding member 8 is configured so as to output at least one of a sound, light, and vibration based on the difference between the measurement data which are acquired through the detecting section 5, and which relate to a body portion of the patient 2, and the reference data which are stored in the storing section 6, and which relate to the body portion.

From the difference between the measurement data and the reference data, for example, it is possible to know the direction along which a body portion of the patient 2 is deviated from a target position. The guiding member 8 performs a motion which suggests the direction opposite to the deviation direction, whereby the body portion of the patient 2 can be guided in the direction along which the deviation is eliminated. From the difference between the measurement data and the reference data, moreover, the distance to the position to which the body portion of the patient 2 is targeted is known. The guiding member 8 performs a motion which corresponds to the distance, whereby the body portion of the patient 2 can be guided in the direction along which the deviation is eliminated.

For example, Fig. 5 illustrates a state where the left hand 2c of the patient 2 is located rightward from the target position indicated by the target position index 3a. At this time, a left side portion of the guiding member 8 vibrates, whereby the patient 2 can recognize that the left hand 2c of the patient must be further leftward moved. The necessity of the leftward movement may be indicated by, in addition to or in place of vibration, lighting of the left side portion, or an output of a sound "Move more leftward." Alternatively, as the left hand 2c is closer to the target position, the vibration or the sound may be changed, or the color, interval, or intensity of the lighting may be changed.

According to the configuration, the patient 2 can know more surely a motion which is to be performed by the patient. Therefore, rehabilitation can be smoothly progressed.

When the guidance by the guiding member 8 is used in combination with the index image illustrated on the first display apparatus 3, the guidance exerts a higher guidance effect. Alternatively, the first display apparatus 3 may not be used, and only the guiding member 8 may perform the guidance. According to the configuration, even a patient who has difficulty in mounting a display apparatus on the head can be caused to easily recognize the target motion, and rehabilitation can be smoothly progressed.

As illustrated in Fig. 1, the assistance system 1 may have a configuration where the system includes a line-of-sight measurement apparatus 9. The line-of-sight measurement apparatus 9 is configured so as to measures the line of sight of the patient 2. The line-of-sight measurement apparatus 9 may be incorporated in the first display apparatus 3, or disposed outside the first display apparatus 3. In this case, the display controller 4 may be configured so as to adjust the position of the index image to be displayed on the first display apparatus 3, based on the line of sight of the patient 2 which is measured by the line-of-sight measurement apparatus 9.

According to the configuration, a control such as that in which an image for guiding to the index image is displayed on a display area of the first display apparatus 3 in accordance with the line of sight which is measured by the line-of-sight measurement apparatus 9 can be performed. For example, the position of the line of sight which is measured by the line-of-sight measurement apparatus 9 may be displayed on the display area of the first display apparatus 3, whereby the patient is caused to recognize the position of the line-of-sight of the patient, and prompted to see the index image. Alternatively, in the case where it is detected that the line-of-sight is located in a place which is separated from the index image (i.e., the patient does not see the index image), an arrow or the like for prompting the guidance to the index image may be displayed on the display area that is in front of the line-of-sight. Therefore, the line-of-sight of the patient can be easily guided to an adequate position. Consequently, rehabilitation can be smoothly progressed.

The line-of-sight of the patient is not required to be specified by direct measurement of the line-of-sight. For example, the line-of-sight of the patient may be indirectly specified by detecting the direction of the face or the upper limb with a sensor or an imaging device. Alco such a configuration is included in "line-of-sight measurement apparatus 9" which is above-described.

As illustrated in Fig. 1, the assistance system 1 may have a configuration where the system includes an evaluating section 10. The evaluating section 10 is configured so as to numerically evaluate the difference between the measurement data which are acquired by the detecting section 5, and which relate to a body portion of the patient 2, and the reference data which are stored in the storing section 6, and which relate to the body portion. An example of the numerical evaluation is the rate of concordance between the target motion indicated by the reference data, and the motion of the patient 2 indicated by the measurement data. For example, several evaluation points are set in a series of motions, and the difference between the measurement data and the reference data at each of the evaluation points is acquired. When the difference at each of the evaluation points is normalized, the rate of concordance of the all motions can be specified. Also differences which are specified at the respective evaluation points may be an example of the numerical evaluation.

The above-described reference data are not limited to data indicating the target motion. For example, the reference data may be data indicating motion in rehabilitation which was performed in past rehabilitation by the patient 2. In the case where the reference data are acquired through the imaging device 52, the rate of concordance between the image related to the reference data, and that related to the measurement data may be evaluated.

According to the configuration, it is possible to quantitatively evaluate the degree by which the motion of the patient 2 coincides with the target motion. Therefore, the medical person can quantitatively evaluate the condition of the patient 2 and an effect of rehabilitation without depending on subjectivity of the observer. Particularly, the setting of a specific target due to quantification contributes to improved patient's motivation for rehabilitation. Consequently, rehabilitation can be smoothly progressed.

As illustrated in Fig. 1, the assistance system 1 may have a configuration where the system includes a second display apparatus 11. The second display apparatus 11 is disposed in a place which is separated from the body of the patient 2. Examples of the second display apparatus 11 are an installation-type display apparatus (such as a display apparatus or a monitor apparatus), and a portable display terminal (such as a smart phone, a tablet terminal, or a head-mounted display). In this case, the display controller 4 may be configured so as to cause a result of evaluation performed by the evaluating section 10 to be displayed on at least one of the first display apparatus 3 and the second display apparatus 11.

According to the configuration, as required, the result of evaluation performed by the evaluating section 10 can be presented on the side of at least one of the patient 2 and the medical person. Namely, the patient and the medical person can share the evaluation result as required. Therefore, the medical person can express an appropriate advice or the like to the patient at an adequate timing. Consequently, sharing of consciousness related to the progress of rehabilitation is prompted, and rehabilitation can be smoothly progressed.

The display controller 4 may have a configuration where the display controller causes an image displayed on the first display apparatus 3 to be displayed also on the second display apparatus 11.

According to the configuration, the medical person can share the scene as viewed from the viewpoint of the patient 2, through the second display apparatus 11. Therefore, the medical person can know easily and intuitively, for example, the difficulty in that the patient 2 follows the target motion. Consequently, the medical person can express an adequate advice or the like to the patient at an appropriate timing. Accordingly, sharing of consciousness related to the progress of rehabilitation is prompted, and rehabilitation can be smoothly progressed.

As illustrated in Fig. 1, the assistance system 1 may have a configuration where the system includes a recording section 12. The recording section 12 is configured so as to record a result of evaluation performed by the evaluating section 10 while associating the result with at least the timing when the evaluation is performed, and patient identification information (the name, the patient number, the date and time of treatment, or the like). Examples of other information to be recorded in the recording section 12 are the name of disease, conditions of treatment, contents of treatment, and the like. Examples of the recording section 12 are a large-capacity storage device (such as a hard disk drive), and a portable storage medium (a disk-type storage medium, a card-type storage medium, a USB memory, or the like). The installation site of the recording section 12 is not particularly limited as far as the section can communicate with the evaluating section 10 in a wired or wireless manner. The recording section may constitute a stand-alone system together with the evaluating section 10, or be connected with the evaluating section 10 via a LAN or a WAN.

According to the configuration, a database of progress situations of rehabilitation of the patient 2 can be produced. Therefore, an effect of rehabilitation is quantitatively evaluated more easily. When an effect of rehabilitation for a long term is quantitatively evaluated, particularly, also an index for determining whether rehabilitation which is appropriate to the patient is performed or not may be set. Therefore, rehabilitation can be smoothly progressed.

The above-described embodiments are examples for facilitating understanding of the invention, and do not limit the invention. The invention may be changed or improved without departing from the spirit of the invention. It is obvious that equivalents are included within the scope of the invention.

In the embodiments, the functions of the display controller 4, the recording image producing section 7, and the evaluating section 10 are realized from software executed by a cooperation of a processor and memory which are communicably connected to each other. Examples of the processor are a CPU and an MPU. Examples of the memory are a RAM and a ROM. However, the function of at least one of the display controller 4, the recording image producing section 7, and the evaluating section 10 may be realized by hardware such as circuit devices, or a combination of hardware and software.

The disclosure of Japanese Patent Application No. 2014-115856 filed June 4, 2014 is incorporated in and constituting part of the description of the present application.

## Claims

1. A rehabilitation assistance system comprising:
a first display apparatus which is to be attached to a head of a patient, and which enables a body portion of the patient to be seen; and
a display controller which causes an index image that functions as an index for a rehabilitation motion, to be displayed on the first display apparatus.

2. The rehabilitation assistance system according to claim 1, wherein the index image contains an image which represents the body portion of the patient.

3. The rehabilitation assistance system according to claim 1 or 2, further comprising:
a detecting section which detects a motion of the body portion of the patient; and
a storing section which previously stores reference data indicating a target motion of the body portion, wherein
the display controller changes a color of the index image based on a difference between data indicating the detected motion and the reference data.

4. The rehabilitation assistance system according to claim 1 or 2, further comprising:
a detecting section which detects a motion of the body portion of the patient; and
a recording image producing section which, based on the motion, produces a recording image indicating the motion of the body portion at a first timing, wherein
the display controller causes the recording image to be displayed on the first display apparatus at a second timing which is later than the first timing.

5. The rehabilitation assistance system according to claim 1 or 2, further comprising:
a detecting section which detects a motion of the body portion of the patient;
a storing section which previously stores reference data indicating a target motion of the body portion; and
a guiding member which is to be attached to the body portion of the patient, and which outputs at least one of a sound, light, and vibration based on a difference between data indicating the detected motion and the reference data.

6. The rehabilitation assistance system according to any one of claims 1 to 5, further comprising
a line-of-sight measurement apparatus which measures a line of sight of the patient, wherein
the display controller adjusts a position of the index image based on the line of sight.

7. The rehabilitation assistance system according to claim 1 or 2, further comprising:
a detecting section which detects a motion of the body portion of the patient;
a storing section which previously stores reference data indicating a motion of the body portion, which functions as a reference; and
an evaluating section which numerically evaluates a difference between data indicating the detected motion and the reference data.

8. The rehabilitation assistance system according to claim 7, further comprising
a second display apparatus which is disposed in a place that is separated from a body of the patient, wherein
the display controller causes a result of evaluation performed by the evaluating section to be displayed on at least one of the first display apparatus and the second display apparatus.

9. The rehabilitation assistance system according to claim 7 or 8, wherein
the display controller causes an image displayed on the first display apparatus to be displayed also on the second display apparatus.

10. The rehabilitation assistance system according to any one of claims 7 to 9, further comprising
a recording section which records a result of evaluation performed by the evaluating section while associating the result of evaluation with at least a timing when the result of evaluation is obtained, and information identifying the patient.

11. A rehabilitation assistance system comprising:
a detecting section which detects a motion of a body portion of a patient;
a storing section which previously stores reference data indicating a target motion of the body portion; and
a guiding member which is to be attached to the body portion of the patient, and which outputs at least one of a sound, light, and vibration based on a difference between data indicating the detected motion and the reference data.
